(19) Europäisches Patentamt
European Patent Office
Office européen des brevets

(11) Publication number: **0 203 787 B1**

# (12) EUROPEAN PATENT SPECIFICATION

(45) Date of publication of patent specification: **30.12.92**

(51) Int. Cl.[5]: **C07D 513/04**, //(C07D513/04, 277:00,235:00),(C07D513/04, 279:00,235:00)

(21) Application number: **86303912.9**

(22) Date of filing: **22.05.86**

## (54) Process for preparing pyridyl-substituted imidazo[2,1-b]thiazoles and thiazines.

(30) Priority: **23.05.85 US 737137**
**12.12.85 US 808595**
**28.04.86 US 856246**

(43) Date of publication of application:
**03.12.86 Bulletin 86/49**

(45) Publication of the grant of the patent:
**30.12.92 Bulletin 92/53**

(84) Designated Contracting States:
**AT BE CH DE FR GB IT LI LU NL SE**

(56) References cited:
**EP-A- 0 000 353**

**CHEMICAL ABSTRACTS, vol. 78, no. 25, 25th June 1973, pages 418,419, abstract no. 159620j, Columbus, Ohio, US; Y. HASHIMOTO et al.: "Imidazo[2,1-b]thiazoline derivatives as pesticidal composites for cattle"**

(73) Proprietor: **SMITHKLINE BEECHAM CORPORATION**
**P.O. Box 7929 1 Franklin Plaza**
**Philadelphia Pennsylvania 19101(US)**

(72) Inventor: **Bender, Paul Elliot**
**504 Lilac Lane**
**Cherry Hill, NJ 08003(US)**
Inventor: **Lantos, Ivan**
**1447 Boxwood Drive**
**Blackwood, NJ 08012(US)**
Inventor: **McGuire, Michael Anthony**
**1334 Harding Boulevard**
**Norristown, PA 19401(US)**
Inventor: **Pridgen, Lendon Norwood**
**805 Morning Dove Road**
**Audubon, PA 19403(US)**
Inventor: **Winicov, Herbert Bernard**
**350 Skippack Pike**
**Blue Bell, PA 19422(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

**CHEMICAL ABSTRACTS, vol. 76, no. 15, 10th April 1972, page 411, abstract no. 85728k, Columbus, Ohio, US; S. KANO: "Thiocyanation of heterocyclic compounds. II. Thiocyanation of imidazo[2,1-b]thiazole derivatives"**

**TETRAHEDRON LETTERS, vol. 23, no. 4, 1982, pages 429-432, Pergamon Press Ltd., GB; KIN-YA AKIBA: "Regioselective synthesis of 4-alkylpyridines via 1,4-dihydropyridine derivatives from pyridine"**

**THE JOURNAL OF ORGANIC CHEMISTRY, vol. 47, no. 22, 22nd October 1982, pages 4315-4319, American Chemical Society; D.L. COMINS et al.: "Regioselective addition of Grignard reagents to 1-acylpyridinium salts. A convenient method for the synthesis of 4-alkyl(aryl)pyridines"**

**YAKUGAKU ZASSHI, vol. 92, no. 1, 1972, pages 51-58; S. KANO: "Studies of thiocyanation of heterocyclic compounds. II. Thiocyanation of imidazo[2,1-b]thiazole derivatives"**

(74) Representative: **Waters, David Martin, Dr. et al**
**Smith Kline & French Laboratories Ltd. Patent Department Mundells**
**Welwyn Garden City Hertfordshire AL7 1EY(GB)**

## Description

This invention relates to new intermediates, processes for preparing them, and their use in the preparation of 5-(4-pyridyl)-6-phenyl-2,3-dihydroimidazo[2,1-b]thiazoles and corresponding thiazines which have activity as inhibitors of the 5-lipoxygenase pathway of arachidonic acid metabolism.

EP-$A_2$-0 000 353 discloses certain anti-inflammatory 1,3-diaza-cyclopent-2-eno[2,1-b]-(1-thia-3-aza-cycloalkanes) and describes methods for their preparation comprising cyclisation of the corresponding open-chain analogues.

Certain anti-arthritic 5,6-diaryl-2,3-dihydroimidazo[2,1-b]thiazoles are described in U.S. Patent No. 4,175,127. The method disclosed therein for preparing them includes a ring closure of the corresponding 4,5-diaryl-2-mercaptoimidazole. If the substituents at the 4,5-positions of the 2-mercaptomidazole are different, the position isomers at the 5,6-positions of the end product 2,3-dihydroimidazo-[2,1-b]thiazoles are produced in about equal quantities. In most cases, and certainly with the preparation of the preferred 5-(4-pyridyl)-6-(4-fluorphenyl)-2,3-dihydroimidazo-[2,1-b]thiazole, the position isomers are separated with difficulty, and even the most efficient separation techniques, preferably using column separation, provide less than 50% yield of the preferred isomer. In practical fact, however, overall yields are much lower. The present invention greatly reduces the cost of chemical for the preparation of the biologically active 5-pyridyl-6-aryl compounds.

Direct addition of various organic moieties to a pyridine ring through the use of Grignard reagents or other organometallic salts is known. For example, it is shown by K. Akiba et al., Tetrahedron Letters, 23, 429 (1982), that 4-alkylpyridines can be prepared with high regioselectivity by reacting a 1-acylpyridinium salt with an alkylcopper-boron trifluoride complex. The synthesis of 1-acyl-4-alkyl(or aryl)-1,4-dihydropyridines by reacting the appropriate alkyl or aryl Grignard reagent with 1-acylpyridinium salt, in the presence of cuprous iodide, is shown in Comins, Tetrahedron Letters, 24, 2807 (1983) and Comins et al, J. Org. Chem., 47, 4315 (1982).

Direct addition of imidazo-thiazole or -thiazine moieties to pyridine, however, has not been previously shown by these methods. The fused-ring Grignard reagents necessary for direct addition to the pyridinium salt have heretofore been unavailable. Nor has it been suggested previously that a 6-phenyl-imidazo[2,1-b]-thiazole (or the corresponding thiazine), by acting as a nucleophile at its 5-position, could bypass the metallic intermediate entirely and add directly to pyridine, particularly with selectivity for the 4-position. The present invention, however, provides methods by which this direct regiospecific addition of the fused-ring moiety can be effected, either by direct nucleophilic attack or by use of novel Grignard intermediates. Accordingly, the present invention avoids the yield loss resulting from the prior art's production of 5,6-position isomers during ring-closure of the corresponding mercaptoimidazole.

In a first aspect, the present invention provides compounds of the formula (I):

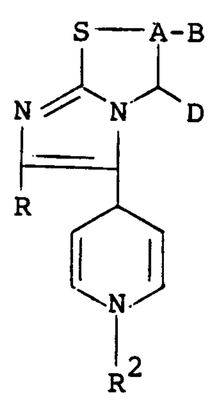

Formula I

in which:

A is methylene or 1,2-ethanediyl;

B and D are independently H, methyl, ethyl, or dimethyl;

R is

(a) phenyl;

(b) monosubstituted phenyl wherein the substituent is halogen, $C_1$-$C_3$ alkoxy, $C_1$-$C_3$ alkylthio, $C_1$-$C_4$ alkyl, $CF_3$, 2,3,2-trihaloethoxy, prop-2-ene-1-oxy, $C_1$-$C_3$ dialkylamino, N-($C_1$-$C_3$ alkyl)-($C_1$-$C_3$ alkanamido), or N-azacycloalkyl of 5 or 6 carbon atoms;

(c) disubstituted phenyl wherein the substituents are independently $C_1$-$C_4$ alkyl or $C_1$-$C_3$ alkoxy, or the substituents together form a methylene-dioxy group; or

(d) 3,4,5-trimethoxyphenyl;

$R^2$ is H or

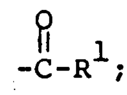

and

$R^1$ is $C_1$-$C_8$ alkyl, $C_1$-$C_8$ alkoxy, phenyl, phenoxy, benzyl, or benzyloxy.

The compounds of the formula (I) are useful as intermediates in the preparation of biologically active final products which are 5-(4-pyridyl)-6-phenyl-2,3-dihydroimidazo[2,1-b]thiazoles and thiazines (hereinafter referred to as "biologically active final products"). All of the biologically active final products have activity as in vivo inhibitors of the 5-lipoxygenase pathway of arachidonic acid metabolism in animals, such as man and other mammals, and some are also useful as intermediates in the preparation of compounds which have activity as in vivo inhibitors of the 5-lipoxygenase pathway in animals such as man and other mammals (as disclosed in EP-231-622-A), and some of the biologically active final products also have activity as inhibitors of the cyclooxygenase pathway of arachidonic acid metabolism, see U.S. Patent No. 4,175,127.

Compounds of Formula I are prepared according to another aspect of this invention by reaction (A):

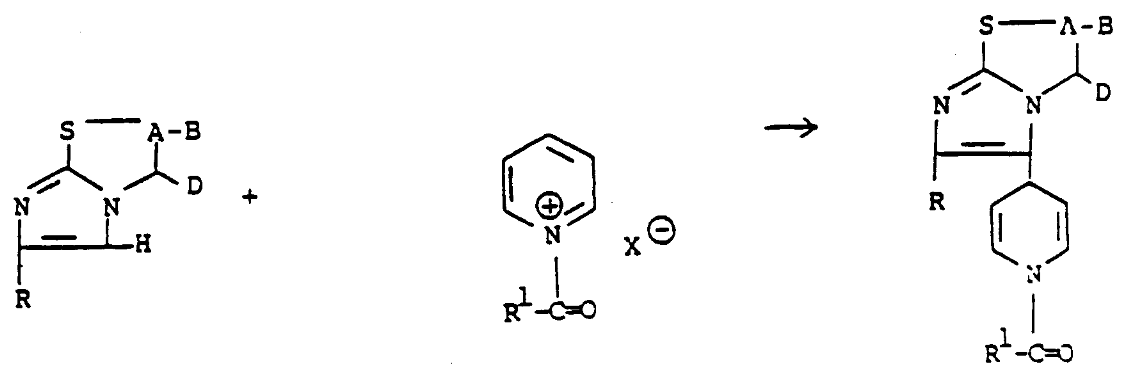

Formula II Formula III Formula I

REACTION (A)

in which:

A, B, D, and R are as described above;

$R^1$ is the non-reactive residue of a reactive acyl ester, especially an acyl halide, such as $C_1$-$C_8$-alkyl, $C_1$-$C_8$-alkoxy, phenyl, phenoxy, benzyl or benzyloxy; and

X is an anion commonly associated with acyl cations or equivalent to the same. Particularly useful are iodide, chloride and bromide. Other anions are acetate, propionate, triflate (trifluoromethylsulfonate), tosylate (p-toluenesulfonate), mesylate (methylsulfonate) or boron tetrafluoride.

The starting materials for this process for Formula II compounds in which A = methylene (thiazole) are described in S. Kano, Yakugaku Zasshi, 92 51-58 (1972), which, in general, describes that 6-phenyl-2,3-dihydroimidazo[2,1-b]thiazoles can be prepared by heating the corresponding 2-amino-4,5-dihydrothiazole with the appropriately substituted phenacyl bromide in an organic solvent, followed by treatment with an alkali such as NaOH to recover the compound from its hydrobromide.

Starting materials for Formula II compounds in which A is 1,2-ethanediyl (thiazine) can be prepared in an analogous manner to the Kano process, described above, from the corresponding 2-amino-5,6-dihydro-4H-1,3-thiazine. More particularly, 7-(4-substituted phenyl)-2,3-dihydro-4H-imidazo [2,1-b]-(1,3)thiazines can be prepared by reacting the corresponding 2-amino-5,6-dihydro-4H-1,3-thiazine with 4-substituted bromoacetophenones in a non-polar solvent such as benzene or chloroform, to form an intermediate which is then refluxed in water. See, Schoeberl, et al., Justus Liebigs Ann. Chem., 742, 85-97 (1970). It has also been found that a polar solvent can be used as well, and that the intermediate may or may not be isolated prior to the refluxing step. The amino-thiazine starting material can be prepared by treating 3-bromopropylamine with t-butyl isothiocyanate, followed by reflux treatment with HBr or HCl. See, for example, Schubert, et al., Arch. Pharm., 301(10), 750-762 (1968).

The reaction (A) is carried out by reacting the 6-arylimidazo[2,1-b]thiazole or thiazine analog of Formula II with an excess of both the pyridine and the reactive acyl ester reactant in an organic solvent in which the reactants are soluble and with which the reactants, especially the acyl ester, are inert. One skilled in the art will recognize that the pyridine and acyl ester react to form the acylpyridinium reagent of Formula III in situ. Optionally, the acylpyridinium reagent can be prepared separately in a solvent and then added to the solution of Formula II compound. Suitable solvents include methylene chloride, ethylene chloride, chloroform, carbon tetrachloride, tetrahydrofuran, ethyl ether, dioxane, toluene, or excess pyridine.

In the conduct of reaction A, the reaction mixture is cooled during the mixing of the reactants, preferably to a temperature between 5-20 ° C, by use of an ice/water bath. The mixture is then allowed to stand briefly at ambient temperature, followed by refluxing until reaction is complete. The reaction mixture is continually assayed using high performance liquid chromatography (HPLC) or thin layer chromatography (TLC) on aliquots to ascertain if unreacted Formula II compound is present. If so, additional acyl pyridinium salt is introduced. Other conditions of the reaction are standard to the art. Following the reaction, the resultant Formula I compounds can be recovered from the reaction mixture and isolated by standard techniques, or the reaction mixture (with no isolation of the Formula I compounds) can be used as the medium for the oxidation step described below as reaction (D).

Compounds of Formula I wherein $R^2$ is

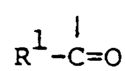

can be prepared by reaction (B):

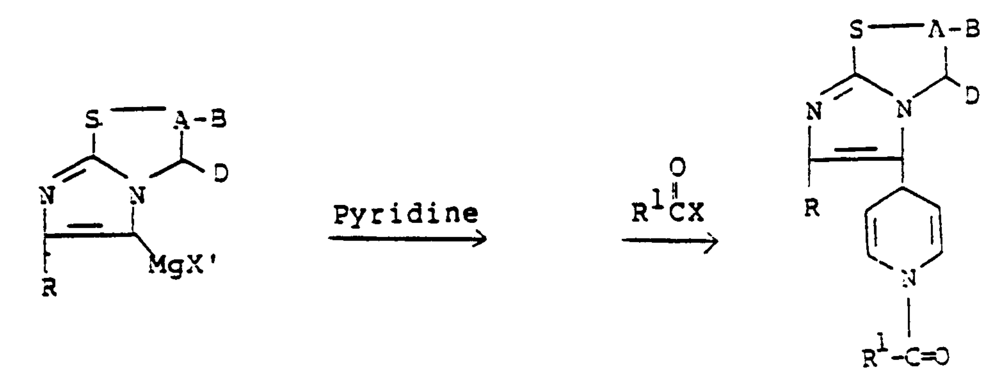


Formula IV

Formula I

REACTION (B)

in which X' is Cl, Br, or I (preferably Br); and R, $R^1$, X, A, B, and D are as described earlier, provided, however, that R is other than N-($C_1$-$C_3$ alkyl)-($C_1$-$C_3$ alkanamido) phenyl.

Reaction (B) is conducted by contacting the Grignard reagent (Formula IV) with an excess of both the

pyridine and the selected reactive acyl ester in a solvent appropriate for Grignard-reagent use. Examples of such solvents include, but are not limited to, dry ethers such as ethyl ether, or preferably, tetrahydrofuran. The Grignard reagent can be combined with the pyridine and the acyl ester in sequence, or as in reaction (A) above, the pyridinium salt can be prepared separately and then added to the solution of Grignard reagent. Preferably, the reaction is conducted by the addition of 2-6 moles of pyridine to each mole of the Grignard reagent, followed by addition of at least an equimolar amount of the acyl compound.

In the conduct of reaction (B), the reactants are mixed into the solvent, with cooling, preferably to a temperature of -10 °C or below. During the mixing period, at least about 5 mole % of cuprous iodide (CuI), based on the molar quantity of pyridine present, is introduced to the reaction mixture to insure the ultimate addition of the Grignard reagent at the 4-position of the N-acyl-1,4-dihydropyridine compound.

When the reactants have been thoroughly dissolved, the reaction initiates autogenously and the mixture can be permitted to warm to a temperature of about 20 °C, but preferably no higher than about 15 °C. Aliquots of the reaction mixture are extracted continuously and assayed using HPLC or TLC to determine the presence of unreacted imidazo[2,1-b]thiazole or thiazine. The conditions of the reaction are otherwise standard for those pertaining to Grignard synthesis in general. As with the Formula I compounds prepared by reaction (A), recovery and isolation can be effected by standard techniques, or the reaction mixture (without isolation of the Formula I compounds) can be used as the medium for the oxidation step of reaction (D) below.

The Grignard reagents of Formula IV are novel compounds. The Formula IV reagents are prepared by reaction (C):

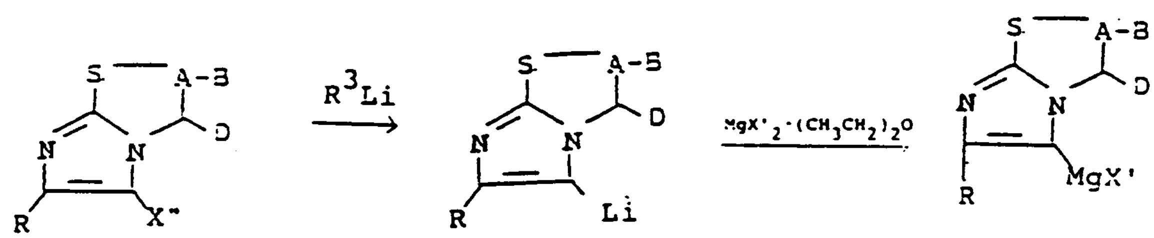


Formula V Formula VI Formula IV

REACTION (C)

in which A, B, D, and R are as earlier defined; X' is Cl, Br, or I, preferably Br; X" is Cl or Br; and $R^3$ is $C_1$-$C_5$ alkyl, preferably butyl and most preferably n-butyl. The reaction (C) is conducted in an ether solvent, preferably tetrahydrofuran, at a temperature below 0 °C. More specifically, the halogenated starting compound of Formula V is reacted with at least an equimolar amount of the alkyllithium compound at a temperature of from about -80 °C to about -30 °C. Following the lithium-halogen exchange, from which the organolithium compound of Formula VI results, the magnesium halide etherate is added to the mixture in excess and reacted with the organolithium compound of Formula VI, during which the reaction temperature can be permitted to rise to a temperature up to about 0 °C, although a reaction temperature below about -10 °C is preferred. The Grignard reagent of Formula IV which is prepared in this manner is preferably used immediately in reaction (B), as described above. This is most easily effected by the addition of cuprous iodide and pyridine, followed by addition of the selected acyl ester to the final reaction mixture of reaction (C).

The starting compounds of Formula V which are used in reaction (C) can be prepared by halogenating the 6-arylimidazo[2,1-b]thiazole or thiazine of Formula II described above. Halogenation is effected by standard techniques, which in general include treating the Formula II compound with $Br_2$ or $Cl_2$ in a neutral solvent at an elevated temperature. For example, 5-bromo-6-phenyl-2,3-dihydroimidazo[2,1-b]thiazole can be prepared by reacting the unbrominated starting material of Formula II with an equimolar amount of $Br_2$ in methylene chloride at reflux temperature. See, S. Kano, Yakagaku Zasshi, 92, 51-58 (1972).

Compounds of the formula (V), with the exception of those wherein A is CH , B and D are H, $X^1$ is Br and R is either 4-bromophenyl or 4-chlorophenyl, form a further aspect of this invention.

Irrespective of which reaction route, (A) or (B), is chosen, the compounds of Formula I are preferably prepared with the most economical pyridinium salt reagents. Examples of such reagents are those in which

$R^1$ is ethoxy, methoxy, methyl, or phenyl.

Overall, the preferred compounds of formula I are as follows:

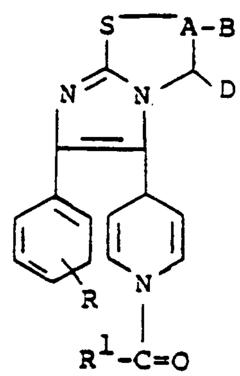

I (Preferred)

in which $R^1$ is ethoxy, methoxy, methyl, or phenyl; A, B, and D are as described above; and R is H, $C_1$-$C_2$ alkyl, $C_1$-$C_2$ alkoxy, trifluoromethyl, N-azacycloalkyl of 5 or 6 carbon atoms, or halogen, preferably F, Cl, or Br. Most preferable are compounds of the preferred class in which B and D are hydrogen, A is -CH-(thiazole), and R is F or Cl, particularly in the 4-position.

In another aspect of the invention, the compounds of Formula I are deacylated and oxidized to form the biologically active final product of Formula VII, as shown in reaction (D):

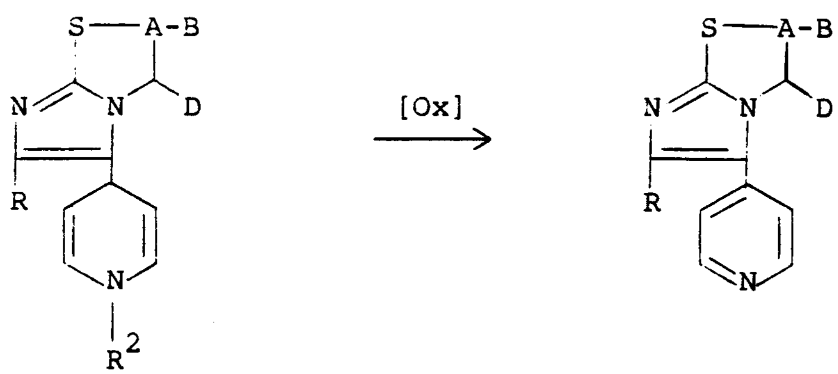


Formula I

Formula VII

REACTION (D)

in which $R^2$ is

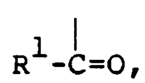

and R, A, B, and D are as defined earlier. Compounds of Formula I in which $R^2$ is hydrogen are intermediates that are formed in situ during the oxidation reaction (D), and as such they are not usually isolated during the reaction sequence, although isolation by standard techniques is possible if desired.

In the conduct of reaction (D), the dihydropyridine of Formula I is reacted with an appropriate oxidizing agent in an inert solvent system until the starting material has been exhausted, as monitored, for example, by periodic TLC or HPLC analysis. Normally, the oxidizing agent is relatively mild, so as to avoid oxidizing the nuclear nitrogen or sulfur members to their respective oxide derivatives. Exemplary oxidation systems are sulfur ($S_8$) in decalin, tetralin, p-cymene, or xylene as solvent; or chloranil, oxygen gas, manganese

oxide, ceric chloride, chromium oxide, hydrogen peroxide or ferricyanide in inert solvents. When the N or S oxide derivatives are the desired end products, a stronger oxidizing agent, such as meta-chloroperbenzoic acid, can be used in excess. Preferred reaction conditions, however, are reaction of a dihydropyridine with sulfur in refluxing xylene or with oxygen in the presence of tert.-butanol/potassium tert.-butoxide.

In general, the oxidation reaction proceeds rapidly, normally within one hour, at a temperature range of from ambient (about 23°C) for oxygen use, to solution reflux temperature, for sulfur use. The oxidized products of formula VII are isolated and purified using methods standard in the art.

## EXAMPLES

The following examples are illustrative, but not limiting of, the operation of this invention. All temperatures are in °C.

## EXAMPLE 1

(A) 6-(4-Fluorophenyl)-2,3-dihydroimidazo[2,1-b]-thiazole (1 mmol, 220 mg) was placed in a clean, dry flask along with a Teflon-coated stir bar. Tetrahydrofuran (10 ml, distilled from sodium/benzophenone) was added by syringe and stirring was begun. Pyridine (1 ml) was added by syringe. The reaction flask was cooled in an ice/water bath. Ethyl chloroformate (6.3 mmol, 0.60 ml) was added by syringe. The pink heterogeneous solution was allowed to stir for 15 hours. The reaction was then heated to 70° for 45 minutes. The reaction was allowed to cool. The solvent was evaporated off in a rotary evaporator. The residue was treated with 10% potassium carbonate solution (25 ml). The mixture was extracted with methylene chloride (2 x 100 ml). The separated methylene chloride phase was dried over anhydrous sodium carbonate, filtered and concentrated in a rotary evaporator. The residue was dissolved in methylene chloride (1 ml) and poured into hexane (50 ml). The resulting heterogeneous solution was filtered and concentrated in a rotary evaporator to give 5-(N-ethoxycarbonyl-1,4-dihydro-4-pyridyl)-6-(4-fluoropheny)-2,3-dihydroimidazo[2,1-b]-thiazole, as a thick oil (80 mg, 22%).

(B) 6-(4-Fluorophenyl)-2,3-dihydroimidazo[2,1-b]-thiazole (2 mmol, 440 mg) was placed in a flask along with a Teflon-coated stir bar. A mild flow of dry nitrogen was begun and continued throughout the reaction. Pyridine (10 mmol, 790 mg) was added to form a slurry. The reaction vessel was cooled in an ice bath for 5 minutes, then ethyl chloroformate (4 mmol, 0.382 ml) was added over a 1 minute period. The solution solidified. The ice bath was removed and replaced by an oil bath. The oil was heated to 75° over a 45 minute period and then allowed to cool. The product was taken up in methylene chloride (100 ml) and extracted with 5% potassium carbonate solution (50 ml). The aqueous layer was back-extracted with methylene chloride (50 ml). The organic layers were combined, dried over sodium sulfate and filtered. The filtrate was concentrated in a rotary evaporator. The resulting oil was purified by silica gel chromatography (1:1, ether/hexane/2% methanol) to yield the 1,4-dihydro compound (300 mg, 40%) as a white crystalline solid, m.p. 139-141°.

Anal. Calcd. for $C_{19}H_{18}N_3O_2SF$: C, 61.44; H, 4.88; N, 11.31. Found: C, 61.32; H, 4.77; N, 11.38.

(C) 6-(4-Fluorophenyl)-2,3-dihydroimidazo[2,1-b]-thiazole (1 mol, 220 g) was placed in a 5L flask along with a large Teflon-coated stir bar. A light flow of dry nitrogen was passed through the system. Methylene chloride (400 ml) was added and stirring was begun. pyridine (11.2 mol, 900 ml) was added all at once. The reaction flask was then placed in a large ice bath and stirred for 10 minutes. Ethyl chloroformate (4.35 mol, 354 ml) was charged into an addition funnel and slow dropwise addition was begun. After 1 hour, the addition was complete. After an additional 30 minutes, the ice bath was removed and the mixture worked up.

Methylene chloride (500 ml) was added. The reaction mixture was extracted with water (3 x 1 ). The organic layer was concentrated on a rotary evaporator to yield the 1,4-dihydropyridine as a brown solid (380 g, 101%). The solid was recrystallized from hot ethanol to yield the desired 1,4-dihydropyridine in 80% yield (300 g).

(D) A mixture of 51.6 g (0.3 mol) of 4-fluorophenacylchloride, 33.66 g (0.33 mol) of 2-aminothiazoline and 200 ml of ethanol was heated at reflux for 2 hours. The mixture was cooled to room temperature at which point 300 ml of water was added. After a reflux period of 2 hours, the mixture was concentrated to remove about 190 ml of aqueous alcohol. The pH of the residue was adjusted to about 2 with 10% hydrochloric acid (10 ml). The solid product was separated and dried to give 65.4 g (85%) of 6-(4-fluorophenyl)-2,3-dihydroimidazo[2,1-b]thiazole, m.p. 116-118°.

HPLC conditions: $C_{18}$-column, methanol/water/monosodium phosphate, 55:45:0.12%.

The procedure of Section D is used to prepare other 5-hydrogen-6-substituted-imidazo[2,1-b]thiazole

starting materials.

EXAMPLE 2

6-(4-Fluorophenyl)-2,3-dihydroimidazo[2,1-b]-thiazole (10.0 g, 0.0456 mol) and 11 ml (0.136 mol) of pyridine (previously stirred over potassium hydroxide) were dissolved in 150 ml of methylene chloride. To this cooled solution (10°) was added 17.75 g (0.13 mol) of isobutylchloroformate in 20 ml of methylene chloride under a nitrogen atmosphere. During the addition, the reaction pot temperature was not allowed to rise past 10°. After the addition, the reddish-colored solution was stirred at ambient temperature for 1 hour then heated under reflux for an additional 15 minutes. Thin-layer chromatography (ethyl acetate/hexane/methanol, 15:1:0.1) detected the presence of starting material. An additional 2.13 g (27 mmol) of pyridine, followed by 3.67 g (27 mmol) of isobutyl chloroformate, was added to the reaction vessel. The solution was stirred at room temperature for 1 hour, then, heated to reflux for 15 minutes. Thin layer analysis at this time did not detect starting material. The reaction mixture was diluted with 200 ml of water and extracted with methylene chloride (3 x 100 ml). The organic layer was dried, then, concentrated to yield 16.0 (85%) of 5-(N-isobutyloxycarbonyl-1,4-dihydro-4-pyridyl)-6-[4-fluoro-phenyl)-2,3-dihydroimidazo[2,1-b] thia zole; m.p. 144-146° (from acetone/hexane).

Calcd. for $C_{21}H_{22}N_3O_2F$: C, 63.14; H, 5.55; N, 10.52. Found: C, 63.27; H, 5.58; N, 10.49.

EXAMPLE 3

6-(4-Fluorophenyl)-2,3-dihydroimidazo[2,1-b]-thiazole(10.0 g, 0.0456 mol) and 11 ml (0.136 mol) of pyridine were dissolved in 150 ml of methylene chloride. To this cooled solution (10°) was added 20.35 g (0.13 mol) of phenyl chloroformate in 20 ml of methylene chloride under a nitrogen atmosphere. During the addition, the reaction pot temperature was not allowed to rise past 15°. After the addition, the reddish-colored solution was stirred at ambient temperature for 1.5 hours, then heated under reflux for an additional 15 minutes. Thin-layer chromatography (ethyl acetate/hexane/methanol, 15:1:0.1) showed the reaction to be complete. The reaction mixture was diluted with 200 ml of water and extracted with methylene chloride (3 x 100 ml). The organic layer was dried, then concentrated to yield 17.0 g (89%) of 5-(N-phenyloxycarbonyl-1,4-dihydro-4-pyridyl)-6-(4-fluorophenyl)-2,3-dihydroimidazo[2,1-b]thiazole; m.p. 164-166° (from acetone/hexane). Calcd. for $C_{23}H_{18}N_3O_2FS$: C, 65.86; H, 4.33; N, 10.02.
Found: C, 64.84; H, 4.41; N, 9.81.

EXAMPLE 4

6-(4-Fluorophenyl)-2,3-dihydroimidazo[2,1-b]thiazole (45.0 g, 0.204 mol) and 55 ml (0.68 mol) of pyridine were dissolved in 500 ml of methylene chloride. To this cooled (ice-water bath) solution (10°) was added 91.3 g (0.65 mol) of benzoyl chloride in 100 ml of methylene chloride under a nitrogen atmosphere. During the addition, the reaction pot temperature was not allowed to rise past 10°. After the addition, the desired product precipitated from solution. The addition of the benzoyl chloride solution was continued. The resulting suspension was stirred at ambient temperature for 15 minutes, then heated to reflux for 15 minutes. Thin-layer chromatography (ethyl acetate/hexane/methanol, 15:1:0.1) showed the presence of some starting material. An additional 20 ml of pyridine followed by benzoyl chloride (28 g, 0.2 mol) were added at room temperature. The suspension was stirred for an additional 0.5 hour. Analysis did not detect starting material at this time. The reaction suspension was filtered. The collected solid was washed with petroleum ether. The solid was vacuum dried to yield 66.8 g (81%) of 5-(N-phenylcarbonyl-1,4-dihydro-4-pyridyl)-6-(4-fluorophenyl)-2,3-dihydroimidazo-[2,1-b]thiazole; m.p. 188-189°.

Calcd. for $C_{23}H_{18}N_3OFS$: C, 68.47; H, 4.50; N, 10.41. Found: C, 68.34; H, 4.61; N, 10.28.

EXAMPLE 5

5-(N-Ethyloxycarbonyl-1,4-dihydro-4-pyridyl)-6-(4-fluorophenyl)-2,3-dihydroimidazo[2,1-b]thiazole (2.5 g, 6.7 mmol) was suspended in 3 ml of p-cymene and heated to 175° for 0.45 hours with 0.3 g (9.4 mmol) of sulfur. Thin-layer chromatography (ethyl acetate/methanol, 9:1) demonstrated the reaction to be complete at this time. Ethyl acetate (100 ml) was added to the dark solution which was then extracted with 10% hydrochloric acid (3 x 50 ml). The acidic layer was made basic with aqueous potassium carbonate and extracted with methylene chloride (3 x 50 ml). The organic layer was, then, dried and concentrated to yield 1.9 g (95%) of crude 5-(4-pyridyl)-6-(4-(fluorophenyl)-2,3-dihydroimidazo[2,1-b]-thiazole. Comparison with

authentic material by TLC was used to verify the identity of the product; m.p. 189°.

EXAMPLE 6

5-(N-Phenylcarbonyl-1,4-dihydro-4-pyridyl)-6-(4-fluorophenyl)-2,3-dihydroimidazo[2,1-b]thiazole (0.5, 1.2 mmol) was dissolved in 2 ml of tert.-butanol containing 0.7 g (6.2 mmol) of potassium tert.-butoxide. Oxygen was bubbled into the solution which was, then, heated under reflux for 15 minutes. The solvent was, then, removed under vacuum and the residue dissolved in methylene chloride. This organic solution was washed with water (2 x 50 ml), dried and concentrated to yield 0.4 g (87%) of crude 5-(4-pyridyl)-6-[4-(fluorophenyl)-]-2,3-dihydroimidazo[2,1-b]thiazole. This material was assayed by HPLC to be 79.5% pure. This reaction was also carried out in aqueous sodium or potassium hydroxide media.

EXAMPLE 7

Using acetyl bromide and 6-(4-chlorophenyl)-2,3-dihydroimidazo[2,1-b]thiazole in the process of Example 5 gives 5-(N-methylcarbonyl-1,4-dihydro-4-pyridyl)-6-(4-chlorophenyl)-2,3-dihydroimidazo[2,1-b] thiazole.

Using benzyl chloroformate and 6-phenyl-2,3-dihydroimidazo[2,1-b]thiazolegives 5-(N-benzyloxycarbonyl-1,4-dihydro-4-pyridyl)-6-phenyl-2,3-dihydroimidazo[2,1-b] thiazole.

Using methyl chloroformate and 6-(2-methylphenyl)-2,3-dihydroimidazo[2,1-b]thiazole gives 5-(N-methoxycarbonyl-1,4-dihydro-4-pyridyl)-6-(2-methylphenyl)-2,3-dihydroimidazo[2,1-b] thiazole.

Using phenylacetyl chloride and 6-(3-methoxyphenyl)-2,3-dihydroimidazo[2,1-b]thiazole gives 5-(N-benzylcarbonyl-1,4-dihydro-4-pyridyl]-6-(3-methoxyphenyl)-2,3-dihydroimidazo[2,1-b]thiazole.

Using the oxidation conditions of Example 6 for each of these dihydropyridyl compounds gives the respective 5-(4-pyridyl)-6-aryl-2,3-dihydroimidazo[2,1-b] thiazoles.

EXAMPLE 8

4-Acetamidoacetophenone (44 g, O.25 mole) was suspended in 500 ml methylene ch]oride and treated with bromine (44 g, 0.275 mole). The reaction mixture was stirred overnight, then evaportated in vacuo. The residue was suspended in 2OO ml of absolute EtOH and treated with 2-amino-4,5-dihydrothiazole (60 g, 0.59 mole). The reaction was stirred for 2 days, then taken up in water and extracted with methylene chloride. The organic phase was washed with water and then brine, and then dried over sodium sulfate. Flash column chromatography (silica, eluting with methylene chloride / methanol, 98/2) gave 6-[4-acetamidopheny])-2,3- dihydroimidazo[2,1-b]thiazole.

The above described acetamide (1O.2 g, O.O39 mole) was hydrolyzed in refluxing 6N HCl (2OO ml) for one hour. The mixture was then cooled and neutralized, and extracted with methylene chloride. The organic layer was washed with brine, dried over sodium sulfate, and evaporated to afford 6.8 g of 6-(4-aminophenyl)-2,3 dihydroimidazo[2,1-b] thiazole.

The above described amine (6.8 g, 0.034 mole) in 150 ml of dimethylformamide was treated with 1,4-dibromobutane (8.4 g, O.O39 mole) and potassium carbonate (15.5 g, O.11 mole). The reaction mixture was stirred at ambient temperature overnight, and the solvent removed in vacuo. The residue was flash chromatographed (silica, methylene chloride / methanol, 97/3) and the crude product recrystallized from methanol to afford O.88 g of 6-(4-(pyrrolidin-1-yl)phenyl-2,3-dihydroimidazo[2,1-b] thiazole, mp 218-22O (dec). A"alyzed for C15 H17 N3 S, Calculated, C: 66.39, H: 6.31, N: 15.48; Found, C: 66.30, H: 6.32, N: 15.27.

The above described pyrrolidine was treated with pyridine and ethyl chloroformate as described in Example 1(B) to afford 5-(N-ethoxycarbonyl 1,1-dihydro-4-pyridyl)-6-(4-(pyrrolidin-1-yl)phenyl-2,3-dihydroimidazo[2,1-b]thiazole.

EXAMPLE 9

5-bromo-6-(4-fluorophenyl)-2,3-dihydroimidazo[1,2-b]thiazole (1.0 mmol, 300 mg, 1 equiv) was weighed into a 50 mL airlessware flask. The flask was fitted with a stir bar and was kept under a constant flow of dry nitrogen. Tetrahydrofuran (THF, 15 mL, freshly distilled from sodium/benzophenone) was added and stirring was begun. The reaction mixture was cooled to -78°C in a dry ice/acetone bath. Butyllithium (1.0 mmol, 2.0 M in hexane) was added via syringe in a dropwise manner. The solution was allowed to warm to -30°C over a 45-minute period, and then held at that temperature for 10 minutes. The solution was then cooled to -50°C, and magnesium bromide etherate (2 mmol, 516 mg. 2 equiv) was added as a solid. The solution

was allowed to warm to 0° C over a 30-minute period and held at that temperature for 15 minutes. The reaction mixture was then cooled to -20° C, copper iodide (20 mg) added, and the mixture maintained at -20° C for 10 minutes, after which dry pyridine (2.0 mmol, 158 mg) was added. After an additional 10 minutes, ethyl chloroformate (1.1 mmol, 119 mg) was added in a dropwise manner. The solution was stirred for 1 hour, during which it was allowed to warm to 20° C. The reaction was then quenched with saturated ammonium chloride/water (2.0 mL) and poured into water (100 mL). The water was extracted with methylene chloride (3 x 50 mL), the organic layer dried over sodium sulfate (anhydrous), and the solvent removed in vacuo to yield a yellow oil which was a mixture of 6-(4-fluorophenyl)-2,3-dihydroimidazo[2,1-b]-thiazole and 5-(N-ethoxycarbonyl-1,4-dihydro-4-pyridyl)-6-(4-fluorophenyl)-2,3-dihydroimidazo[2,1-b]thiazole (260 mg of oil). The latter compound can be purified by silica gel chromatography (solvent = ether/hexane) to provide a 30-40% yield.

EXAMPLE 10

Using acetyl bromide and 5-bromo-6-(4-chlorophenyl)-2,3-dihydroimidazo[2,1-b]thiazole in the process of Example 9 gives 5-(N-methylcarbonyl-1,4-dihydro-4-pyridyl)-6-(4-chlorophenyl)-2,3-dihydroimidazo[2,1 - b]thiazole.

Using benzyl chloroformate and 5-chloro-6-phenyl-2,3-dihydroimidazo[2,1-b]thiazole gives 5-(N-benzyloxycarbonyl-1,4-dihydro-4-pyridyl)-6-phenyl-2,3-dihydroimidazo[2,1-b]thiazole.

Using methyl chloroformate and 5-bromo-6-(2-methyl-phenyl)-2,3-dihydroimidazo[2,1-b]thiazole gives 5-(N-methoxycarbonyl-1,4-dihydro-4-pyridyl)-6-(2-methylphenyl)-2,3-dihydroimidazo[2,1-b]thiazole.

Using 6-bromo-7-(4-fluorophenyl)-2,3-dihydro-4H-imidazo[2,1-b]-)1,3)thiazine in the process of Example 9 gives 6-(N-ethoxycarbonyl-1,4-dihydro-4-pyridyl)-7-(4-fluorophenyl)-2,3-dihydro-4H-imidazo[2,1-b] - (1,3)-thiazine.

## Claims

**Claims for the following Contracting States : BE, CH, DE, FR, GB, IT, LI, LU, NL, SE**

**1.** A compound of the formula

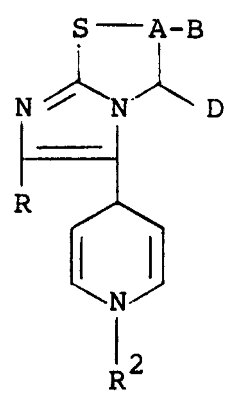

in which:

A is methylene or 1,2-ethanediyl;

B and D are independently H, methyl, ethyl, or dimethyl;

R is

(a) phenyl;

(b) monosubstituted phenyl wherein the substituent is halogen, $C_1$-$C_3$ alkoxy, $C_1$-$C_3$ alkylthio, $C_1$-$C_4$ alkyl, $CF_3$, 2,2,2-trihaloethoxy, prop-2-ene-1-oxy, $C_1$-$C_3$ dialkylamino, N-($C_1$-$C_3$ alkyl)-($C_1$-$C_3$ alkanamido), or N-azacycloalkyl of 5 or 6 carbon atoms;

(c) disubstituted phenyl wherein the substituents are independently $C_1$-$C_4$ alkyl or $C_1$-$C_3$ alkoxy, or the substituents together form a methylene-dioxy group; or

(d) 3,4,5-trimethoxyphenyl;

$R^2$ is H or

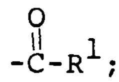

and

$R^1$ is $C_1$-$C_8$ alkyl, $C_1$-$C_8$ alkoxy, phenyl, phenoxy, benzyl, or benzyloxy.

**2.** A compound according to Claim 1 in which R is phenyl or phenyl monosubstituted with halogen, $C_1$-$C_2$ alkyl, $C_1$-$C_2$ alkoxy, $CF_3$, or N-azacyloalkyl of 5 or 6 carbon atoms.

**3.** A compound according to Claim 1 in which R is 4-fluorophenyl or 4-chlorophenyl.

**4.** A compound according to Claim 1 having the structure

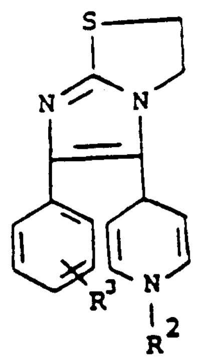

in which:

$R^3$ is hydrogen, halo, $C_1$-$C_2$-alkyl, $C_1$-$C_2$-alkoxy or trifluoromethyl;

$R^2$ is H or

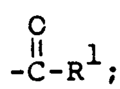

and

$R^1$ is $C_1$-$C_8$-alkyl, $C_1$-$C_8$-alkoxy, phenyl, phenoxy, benzyl or benzyloxy.

**5.** A compound according to Claim 4 in which $R^3$ is 4-fluoro.

**6.** A compound according to any one of claims 1 to 5 in which $R^2$ is

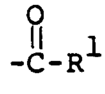

and $R^1$ is ethoxy, methoxy, methyl or phenyl.

**7.** A process for preparing a compound of the formula

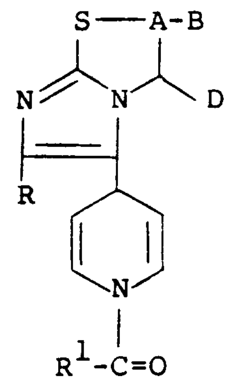

in which A, B, D, R and $R^1$ are as defined in claim 1; comprising reacting a compound of the formula

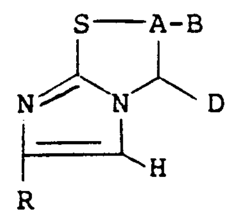

in which A, B, D and R are as defined above, with an N-acylpyridinium salt of the formula:

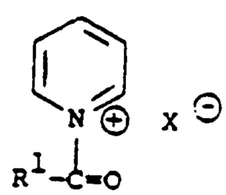

in which $R^1$ is as defined above, and X is an anion commonly associated with acyl cations.

**8.** A process for preparing a compound of the formula

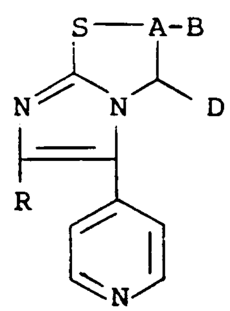

in which A, B, D and R are as defined in any one of claims 1 to 3; comprising reacting a compound as defined in any one of claims 1 to 3 with an oxidising agent.

**9.** The method of Claim 8 in which the oxidizing agent is oxygen or sulphur.

**10.** The method of Claim 9 in which the oxidizing agent is oxygen in the presence of t-butanol or potassium t-butoxide.

**Claims for the following Contracting State : AT**

**1.** A process of preparing a compound of the formula

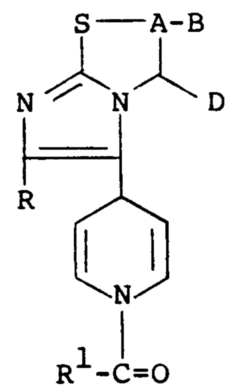

in which
A is methylene or 1,2-ethanediyl;
B and D are independently H, methyl, ethyl, or dimethyl;
R is
(a) phenyl;
(b) monosubstituted phenyl wherein the substituent is halogen, $C_1$-$C_3$ alkoxy, $C_1$-$C_3$ alkylthio, $C_1$-$C_4$ alkyl, $CF_3$, 2,2,2-trihaloethoxy, prop-2-ene-1-oxy, $C_1$-$C_3$ dialkylamino, N-($C_1$-$C_3$ alkyl)-($C_1$-$C_3$ alkanamido), or N-azacycloalkyl of 5 or 6 carbon atoms;
(c) disubstituted phenyl wherein the substituents are independently $C_1$-$C_4$ alkyl or $C_1$-$C_3$ alkoxy, or the substituents together form a methylene-dioxy group; or
(d) 3,4,5-trimethoxyphenyl; and
$R^1$ is $C_1$-$C_8$ alkyl, $C_1$-$C_8$ alkoxy, phenyl, phenoxy, benzyl, or benzyloxy;
comprising reacting a compound of the formula

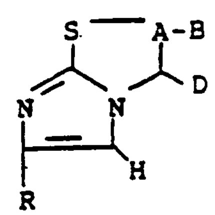

in which A, B, D and R are as defined above, with an N-acylpyridinium salt of the formula:

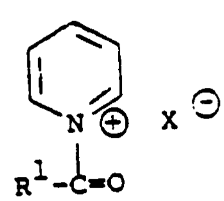

in which $R^1$ is as defined above, and X is an anion commonly associated with acyl cations.

**2.** The process of Claim 1 in which R is phenyl or phenyl monosubstituted with halogen, $C_1$-$C_2$ alkyl, $C_1$-$C_2$ alkoxy, N-azacycloalkyl of 5 or 6 carbon atoms, or $CF_3$; and in which X is bromo, chloro, or iodo.

**3.** The process of Claim 2 in which R is 4-fluorophenyl and in which X is bromo, chloro, or iodo.

**4.** The process of Claim 3 in which $R^1$ is ethoxy, methoxy, methyl, or phenyl.

**5.** A process according to claim 1 for preparing a compound of the formula

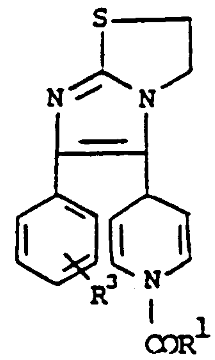

in which:
$R^3$ is hydrogen, halo, $C_1$-$C_2$-alkyl, $C_1$-$C_2$-alkoxy or trifluoromethyl; and
$R^1$ is $C_1$-$C_8$-alkyl, $C_1$-$C_8$-alkoxy,
phenyl, phenoxy, benzyl or benzyloxy.

**6.** A process for preparing a compound of the formula

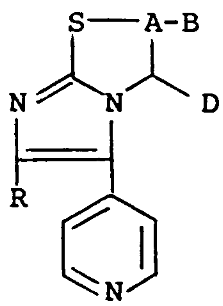

in which A, B, D and R are as defined in claim 1; comprising reacting a compound of the formula

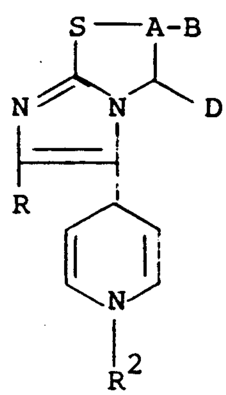

in which;
A,B,D, and R are as defined above;

$R^2$ is H or

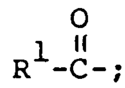

and

$R^1$ is $C_1$-$C_8$ alkoxy, phenyl, phenoxy, benzyl, or benzyloxy,

with an oxidizing agent.

**7.** The method of Claim 6 in which R is phenyl or phenyl monosubstituted with halogen, $C_1$-$C_2$ alkyl, $C_1$-$C_2$ alkoxy, $CF_3$, or N-azacycloalkyl of 5 or 6 carbon atoms.

**8.** The method of Claim 7 in which the oxidizing agent is oxygen or sulphur.

**9.** The method of Claim 8 in which the oxidizing agent is oxygen in the presence of t-butanol or potassium t-butoxide.

**Patentansprüche**
**Patentansprüche für folgende Vertragsstaaten : BE, CH, DE, FR, GB, IT, LI, LU, NL, SE**

**1.** Verbindung der Formel

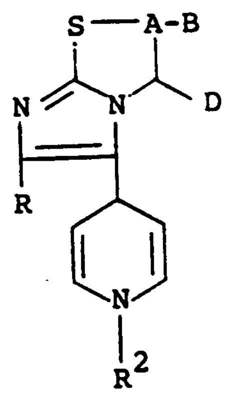

in der

A eine Methylen- oder 1,2-Ethandiylgruppe ist;

B und D unabhängig ein Wasserstoffatom, eine Methyl-, Ethyl- oder Dimethylgruppe sind;

der Rest R

(a) eine Phenylgruppe ist;

(b) eine monosubstituierte Phenylgruppe ist, wobei der Substituent ein Halogenatom, ein $C_{1-3}$-Alkoxy-, $C_{1-3}$-Alkylthio-, $C_{1-4}$-Alkylrest, eine $CF_3$-Gruppe, ein 2,2,2-Trihaloethoxyrest, eine Prop-2-en-1-oxygruppe, ein $C_{1-3}$-Dialkylamino-, N-($C_{1-3}$-Alkyl)-($C_{1-3}$-alkanamido)- oder N-Azacycloalkylrest mit 5 oder 6 Kohlenstoffatomen ist;

(c) ein disubstituierter Phenylrest ist, wobei die Substituenten unabhängig $C_{1-4}$-Alkyl- oder $C_{1-3}$-Alkoxyreste sind oder die Substituenten zusammen eine Methylendioxygruppe bilden; oder

(d) eine 3,4,5-Trimethoxyphenylgruppe ist;

$R^2$ ein Wasserstoffatom oder ein Rest

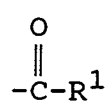

ist;und

$R^1$ ein $C_{1-8}$-Alkyl-, $C_{1-8}$-Alkoxyrest, eine Phenyl-, Phenoxy-, Benzyl- oder Benzyloxygruppe ist.

**2.** Verbindung gemäß Anspruch 1, in der R eine Phenylgruppe oder eine mit einem Halogenatom, einem $C_{1-2}$-Alkyl-, $C_{1-2}$-Alkoxyrest, einer $CF_3$-Gruppe oder einem N-Azacycloalkylrest mit 5 oder 6 Kohlenstoffatomen monosubstituierte Phenylgruppe ist.

**3.** Verbindung gemäß Anspruch 1, in der R eine 4-Fluorphenyl- oder 4-Chlorphenylgruppe ist.

**4.** Verbindung gemäß Anspruch 1 mit der Struktur

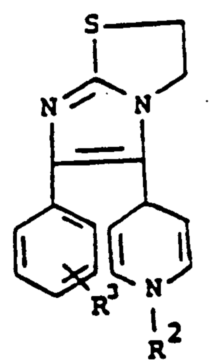

in der
$R^3$ ein Wasserstoff-, Halogenatom, ein $C_{1-2}$-Alkyl-, $C_{1-2}$-Alkoxyrest oder eine Trifluormethylgruppe ist;
$R^2$ ein Wasserstoffatom oder ein Rest

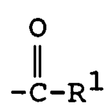

ist; und
$R^1$ ein $C_{1-8}$-Alkyl-, $C_{1-8}$-Alkoxyrest, eine Phenyl-, Phenoxy-, Benzyl- oder Benzyloxygruppe ist.

**5.** Verbindung gemäß Anspruch 4, in der $R^3$ ein 4-Fluoratom ist.

**6.** Verbindung gemäß einem der Ansprüche 1 bis 5, in der $R^2$ ein Rest

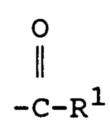

ist und $R^1$ eine Ethoxy-, Methoxy-, Methyl- oder Phenylgruppe ist.

**7.** Verfahren zur Herstellung einer Verbindung der Formel

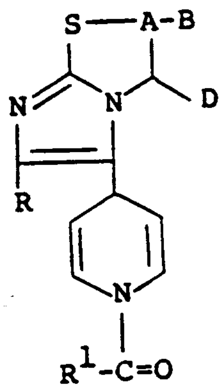

in der A, B, D, R und $R^1$ wie in Anspruch 1 definiert sind, das die Umsetzung einer Verbindung der Formel

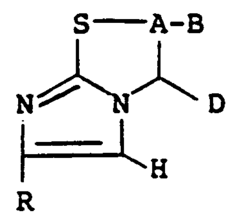

in der A, B, D und R wie vorstehend definiert sind, mit einem N-Acylpyridiniumsalz der Formel

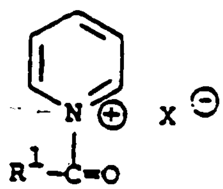

umfaßt, in der $R^1$ wie vorstehend definiert ist und X ein Anion ist, das üblicherweise mit Acylkationen verbunden ist.

**8.** Verfahren zur Herstellung einer Verbindung der Formel

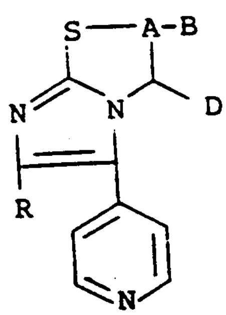

in der A, B, D und R wie in einem der Ansprüche 1 bis 3 definiert sind, das die Umsetzung einer Verbindung, wie sie in einem der Ansprüche 1 bis 3 definiert ist, mit einem Oxidationsmittel umfaßt.

**9.** Verfahren nach Anspruch 8, in dem das Oxidationsmittel Sauerstoff oder Schwefel ist.

**10.** Verfahren nach Anspruch 9, in dem das Oxidationsmittel Sauerstoff in Gegenwart von t-Butanol oder

Kalium-t-butoxid ist.

**Patentansprüche für folgenden Vertragsstaat : AT**

1. Verfahren zur Herstellung einer Verbindung der Formel

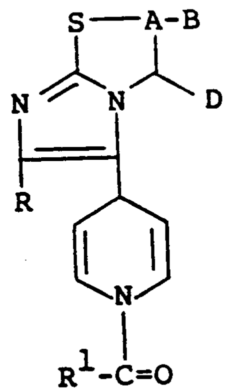

in der
A eine Methylen- oder 1,2-Ethandiylgruppe ist;
B und D unabhängig ein Wasserstoffatom, eine Methyl-, Ethyl- oder Dimethylgruppe sind;
der Rest R
(a) eine Phenylgruppe ist;
(b) eine monosubstituierte Phenylgruppe ist, wobei der Substituent ein Halogenatom, ein $C_{1-3}$-Alkoxy-, $C_{1-3}$-Alkylthio-, $C_{1-4}$-Alkylrest, eine $CF_3$-Gruppe, ein 2,2,2-Trihaloethoxyrest, eine Prop-2-en-1-oxygruppe, ein $C_{1-3}$-Dialkylamino-, N-($C_{1-3}$-Alkyl)-($C_{1-3}$-alkanamido)- oder N-Azacycloalkylrest mit 5 oder 6 Kohlenstoffatomen ist;
(c) ein disubstituierter Phenylrest ist, wobei die Substituenten unabhängig $C_{1-4}$-Alkyl- oder $C_{1-3}$-Alkoxyreste sind oder die Substituenten zusammen eine Methylendioxygruppe bilden; oder
(d) eine 3,4,5-Trimethoxyphenylgruppe ist; und
$R^1$ ein $C_{1-8}$-Alkyl-, $C_{1-8}$-Alkoxyrest, eine Phenyl-, Phenoxy-, Benzyl- oder Benzyloxygruppe ist, das die Umsetzung einer Verbindung der Formel

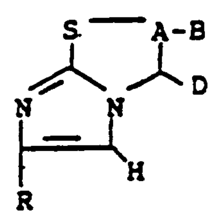

in der A, B, D und R wie vorstehend definiert sind, mit einem N-Acylpyridiniumsalz der Formel

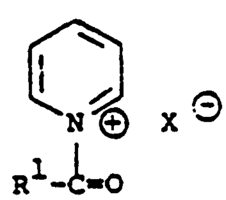

umfaßt, in der $R^1$ wie vorstehend definiert ist und X ein Anion ist, das üblicherweise mit Acylkationen verbunden ist.

2. Verfahren gemäß Anspruch 1, bei dem R eine Phenylgruppe oder eine mit einem Halogenatom, einem $C_{1-2}$-Alkyl-, $C_{1-2}$-Alkoxyrest, einer $CF_3$-Gruppe oder einem N-Azacycloalkylrest mit 5 oder 6 Kohlenstoffatomen monosubstituierte Phenylgruppe ist und in der X Brom, Chlor oder Jod ist.

3. Verfahren gemäß Anspruch 2, bei dem R eine 4-Fluorphenylgruppe ist und X Brom, Chlor oder Jod ist.

4. Verfahren gemäß Anspruch 3, bei dem $R^1$ eine Ethoxy-, Methoxy-, Methyl- oder Phenylgruppe ist.

5. Verfahren gemäß Anspruch 1 zur Herstellung einer Verbindung der Formel

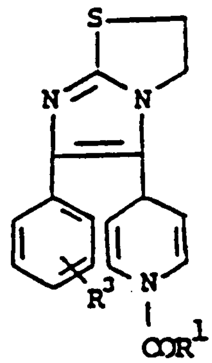

in der
$R^3$ ein Wasserstoff-, Halogenatom, ein $C_{1-2}$-Alkyl-, $C_{1-2}$-Alkoxyrest oder eine Trifluormethylgruppe ist; und
$R^1$ ein $C_{1-8}$-Alkyl-, $C_{1-8}$-Alkoxyrest, eine Phenyl-, Phenoxy-, Benzyl- oder Benzyloxygruppe ist.

6. Verfahren zur Herstellung einer Verbindung der Formel

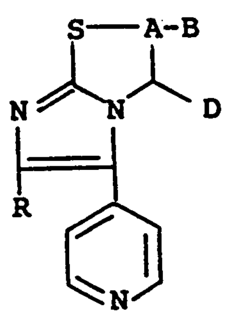

in der A, B, D und R wie in Anspruch 1 definiert sind, das die Umsetzung einer Verbindung der Formel

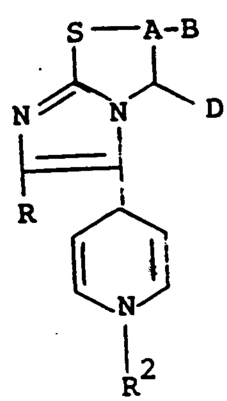

in der A, B und R wie vorstehend definiert sind, $R^2$ ein Wasserstoffatom oder einen Rest

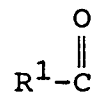

bedeutet, und
$R^1$ ein $C_{1-8}$-Alkoxyrest, eine Phenyl-, Phenoxy-, Benzyl- oder Benzyloxygruppe ist,
mit einem Oxidationsmittel umfaßt.

**7.** Verfahren gemäß Anspruch 6, bei dem R eine Phenylgruppe oder eine mit einem Halogenatom, einem $C_{1-2}$-Alkyl-, $C_{1-2}$-Alkoxyrest, einer $CF_3$-Gruppe oder einem N-Azacycloalkylrest mit 5 oder 6 Kohlenstoffatomen monosubstituierte Phenylgruppe ist.

**8.** Verfahren nach Anspruch 7, in dem das Oxidationsmittel Sauerstoff oder Schwefel ist.

**9.** Verfahren nach Anspruch 8, in dem das Oxidationsmittel Sauerstoff in Gegenwart von t-Butanol oder Kalium-t-butoxid ist.

**Revendications**
**Revendications pour les Etats contractants suivants : BE, CH, DE, FR, GB, IT, LI, LU, NL, SE**

**1.** Composé de formule :

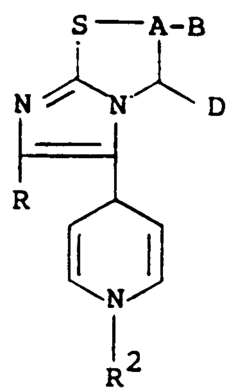

dans laquelle
A est un groupe méthylène ou 1,2-éthanediyle ;
B et D sont indépendamment un atome d'hydrogène, un groupe méthyle, éthyle ou diméthyle ;
R est
(a) un groupe phényle ;
(b) un groupe phényle monosubstitué dans lequel le substituant est un atome d'halogène, un groupe alcoxy en $C_1$-$C_3$ , alkylthio en $C_1$-$C_3$, alkyle en $C_1$-$C_4$ , $CF_3$ , 2,2,2-trihaloéthoxy, prop-2-ène-1-oxy, dialkylamino en $C_1$-$C_3$ , N-(alkyle en $C_1$-$C_3$)-alcanamido en $C_1$-$C_3$ ) ou N-azacycloalkyle de 5 ou 6 atomes de carbone ;
(c) un groupe phényle disubstitué dans lequel les substituants sont indépendamment un groupe alkyle en $C_1$-$C_4$ ou alcoxy en $C_1$-$C_3$ ,ou les substituants forment ensemble un groupe méthylènedioxy ; ou
(d) un groupe 3,4,5-triméthoxyphényle ;
$R^2$ est un atome d'hydrogène ou un groupe

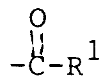

; et
$R^1$ est un groupe alkyle en $C_1$-$C_8$, alcoxy $C_1$-$C_8$, phényle, phénoxy, benzyle ou benzyloxy.

**2.** Composé suivant la revendication 1, dans lequel R est un groupe phényle ou phényle monosubstitué par un atome d'halogène, un groupe alkyle en $C_1$-$C_2$ , alcoxy en $C_1$-$C_2$ , $CF_3$ ou N-azacycloalkyle de 5 ou 6 atomes de carbone.

**3.** Composé suivant la revendication 1 dans lequel R est un groupe 4-fluorophényle ou 4-chlorophényle.

**4.** Composé suivant la revendication 1, ayant la structure :

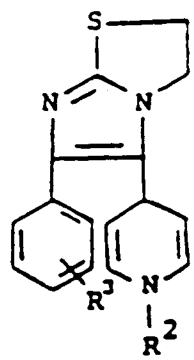

dans laquelle
$R^3$ est un atome d'hydrogène, d'halogène, un groupe alkyle en $C_1$-$C_2$ , alcoxy en $C_1$-$C_2$ ou trifluorométhyle ;
$R^2$ est un atome d'hydrogène ou un groupe

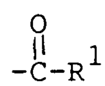

; et
$R^1$ est un groupe alkyle en $C_1$-$C_8$ , alcoxy en $C_1$-$C_8$ , phényle, phénoxy, benzyle ou benzyloxy.

**5.** Composé suivant la revendication 4, dans lequel $R^3$ est un groupe 4-fluoro.

**6.** Composé suivant l'une quelconque des revendications 1 à 5, dans lequel $R^2$ est

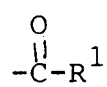

et $R^1$ est un groupe éthoxy, méthoxy, méthyle ou phényle.

**7.** Procédé pour préparer un composé de formule :

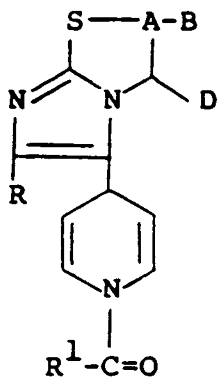

dans laquelle
A, B, D, R et $R^1$ sont tels que définis dans la revendication 1 , comprenant la réaction d'un composé de formule :

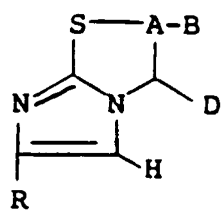

dans laquelle
A, B, D et R sont tels que définis plus haut, avec un sel de N-acylpyridinium de formule :

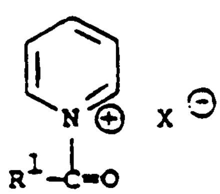

dans laquelle
$R^1$ est tel que défini plus haut, et
X est un anion couramment associé avec des cations acyle.

**8.** Procédé pour préparer un composé de formule :

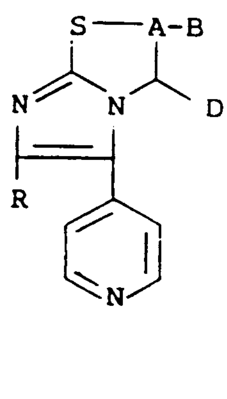

dans laquelle

A, B, D et R sont tels que définis dans l'une quelconque des revendications 1 à 3, comprenant la réaction d'un composé suivant l'une quelconque des revendications 1 à 3 avec un agent oxydant.

**9.** Procédé suivant la revendication 8, dans lequel l'agent oxydant est de l'oxygène ou du soufre.

**10.** Procédé suivant la revendication 9, dans lequel l'agent oxydant est de l'oxygène en présence de t-butanol ou de t-butylate de potassium.

**Revendications pour l'Etat contractant suivant : AT**

**1.** Procédé pour préparer un composé de formule :

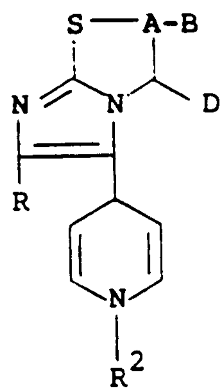

dans laquelle
A est un groupe méthylène ou 1,2-éthanediyle ;
B et D sont indépendamment un atome d'hydrogène, un groupe méthyle, éthyle ou diméthyle ;
R est
(a) un groupe phényle ;
(b) un groupe phényle monosubstitué dans lequel le substituant est un atome d'halogène, un groupe alcoxy en $C_1$-$C_3$ , alkylthio en $C_1$-$C_3$, alkyle en $C_1$-$C_4$ , $CF_3$ , 2,2,2-trihaloéthoxy, prop-2-ène-1-oxy, dialkylamino en $C_1$-$C_3$ , N-(alkyle en $C_1$-$C_3$)-alcanamido en $C_1$-$C_3$ ) ou N-azacycloalkyle de 5 ou 6 atomes de carbone ;
(c) un groupe phényle disubstitué dans lequel les substituants sont indépendamment un groupe alkyle en $C_1$-$C_4$ ou alcoxy en $C_1$-$C_3$ ,ou les substituants forment ensemble un groupe méthylènedioxy ; ou
(d) un groupe 3,4,5-triméthoxyphényle ;
$R^2$ est un atome d'hydrogène ou un groupe

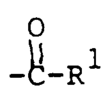 ;

et
$R^1$ est un groupe alkyle en $C_1$-$C_8$, alcoxy $C_1$-$C_8$, phényle, phénoxy, benzyle ou benzyloxy. comprenant la réaction d'un composé de formule :

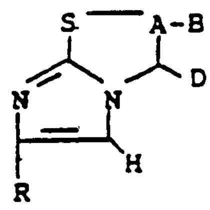

dans laquelle
A, B, D et R sont tels que définis plus haut, avec un sel de N-acylpyridinium de formule :

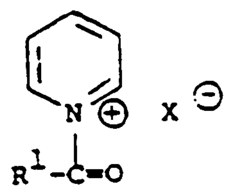

dans laquelle
$R^1$ est tel que défini plus haut ; et
X est un anion couramment associé avec des cations acyle.

**2.** Procédé suivant la revendication 1, dans lequel R est un groupe phényle ou phényle monosubstitué par un atome d'halogène, un groupe alkyle en $C_1$-$C_2$ , alcoxy en $C_1$-$C_2$ , N-azacycloalkyle de 5 ou 6 atomes de carbone, ou $CF_3$ ; et dans lequel X est un atome de brome, de chlore ou d'iode.

**3.** Procédé suivant la revendication 2, dans lequel R est un groupe 4-fluorophényle et X est un atome de brome, de chlore ou d'iode.

**4.** Procédé suivant la revendication 3, dans lequel $R^1$ est un groupe éthoxy, méthoxy, méthyle ou phényle.

**5.** Procédé suivant la revendication 1, pour préparer un composé de formule :

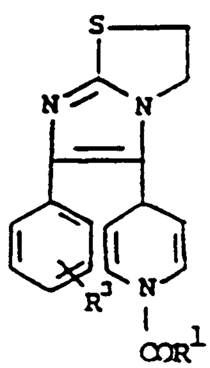

dans laquelle
$R^3$ est un atome d'hydrogène, d'halogène, un groupe alkyle en $C_1$-$C_2$ , alcoxy en $C_1$-$C_2$ ou trifluorométhyle ; et
$R^1$ est un groupe alkyle en $C_1$-$C_8$, alcoxy en $C_1$-$C_8$, phényle, phénoxy, benzyle ou benzyloxy.

**6.** Procédé pour préparer un composé de formule :

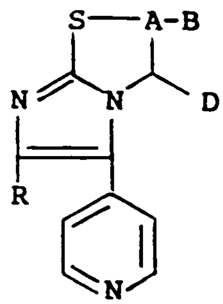

dans laquelle A, B, D et R sont tels que définis dans la revendication 1, comprenant la réaction d'un composé de formule :

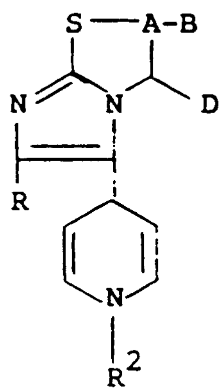

dans laquelle
A, B, D et R sont tels que définis plus haut ;
$R^2$ est un atome d'hydrogène ou un groupe

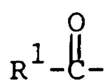 ;

et
$R^1$ est un groupe alcoxy en $C_1$-$C_8$ , phényle, phénoxy, benzyle ou benzyloxy,
avec un agent oxydant.

**7.** Procédé suivant la revendication 6, dans lequel R est un groupe phényle ou phényle monosubstitué avec un atome d'halogène, un groupe alkyle en $C_1$-$C_2$ , alcoxy en $C_1$-$C_2$ , $CF_3$ , ou N-azacycloalkyle de 5 ou 6 atomes de carbone.

**8.** Procédé suivant la revendication 7, dans lequel l'agent oxydant est de l'oxygène ou du soufre.

**9.** Procédé suivant la revendication 8, dans lequel l'agent oxydant est de l'oxygène en présence de t-butanol ou de t-butylate de potassium.